# EUROPÄISCHE PATENTSCHRIFT

(11) **EP 2 394 684 B1**
(45) Veröffentlichungstag und Bekanntmachung des Hinweises auf die Patenterteilung: **09.09.2015**
(21) Anmeldenummer: 10012851.1
(22) Anmeldetag: 26.02.2002
(51) Int. Cl.: A61M 5/32, A61M 25/02, A61B 17/34, A61M 25/06

(54) **Injektionsnadel mit Schutzelement für die Nadelspitze**
Injection needle with a protection element for the needle tip
Aiguille d'injection avec un élément de protection pour la pointe de l'aiguille

(30) Priorität: 26.02.2001 DE 20103363 U
(43) Veröffentlichungstag der Anmeldung: 14.12.2011
(62) Teilanmeldung aus: 06013171.1
(73) Patentinhaber: B. Braun Melsungen AG, 34212 Melsungen (DE)
(72) Erfinder: Woehr, Kevin, 34587 Felsberg (DE); Fuchs, Jürgen, 34308 Bad Emstal (DE)
(74) Vertreter: Kinkeldey, Daniela

(56) Entgegenhaltungen:
- EP-A- 0 554 841
- WO-A-99/08742
- WO-A1-00/69501
- US-A- 5 344 408
- US-A- 5 584 810

## Beschreibung

Die Erfindung betrifft eine Schutzvorrichtung für eine Injektions bzw. Infusionsnadel nach dem Oberbegriff des Anspruchs 1. Eine derartige Schutzvorrichtung ist aus der EP 0 554 841 A1 bekannt.

Eine ähnliche Schutzvorrichtung ist beispielsweise aus US 4 929 241 bekannt, wobei ein retativ kleines Schutzelement auf der Nadel angeordnet ist, das mittels einer Feder aus der zurückgezogenen Stellung in die Schutzstellung an der Nadelspitze-verschoben werden kann, wobei elastische Arme des Schutzelementes die Nadelspitze übergreifen, während eine Eingriffseinrichtung am Schutzelement dieses auf dem Nadelschaft hält. Aufgrund der geringen Größe des Schutzelementes ist es schwierig, dieses von Hand auf der Nadel zu verschieben. Zudem kann die Befestigungsfeder erst dann ausgelöst werden, wenn die Nadelspitze frei liegt, so dass eine Verletzungsgefahr nicht ausgeschlossen werden kann.

Der Erfindung liegt die Aufgabe zugrunde, eine Schutzvorrichtung der eingangs angegebenen Art so auszubilden, dass eine Betätigung von Hand erleichtert wird und eine Verletzungsgefahr ausgeschlossen werden kann.

Diese Aufgabe wird erfindungsgemäß durch die Merkmale im kennzeichnenden Teil des Anspruchs 1 gelöst. Dadurch, dass ein Griffteil zwischen Schutzelement und Nadelhalter zum Verschieben des Schutzelementes vorgesehen ist, kann beim Zurückziehen der Nadel mittels des Nadelhalters nach einer Injektion mit der anderen Hand das Griffteil bequem gehalten werden, so dass durch die Relativbewegung zwischen Nadel und Griffteil das Schutzelement in die Schutzposition an der Nadelspitze verschoben wird, ohne dass das kleine Schutzele ment mit den Fingern berührt werden muss. Dadurch, dass das Schutzelement während des Zurückziehens der Nadel in die Schutzposition gebracht wird, kann auch eine Verletzungsgefahr ausgeschlossen werden. Desweiteren weist das Schutzelement zwei sich Krenzende Arme mit abgewinkelten Enden auf, die die Nadelspitze in der Schutzstellung übergreifen.

Beispielsweise Ausfubrungsfonnen der Erfindung werden nachfolgend anhand der Zeichnungen näher erläutert. Es zeigen
- Fig. 1: in einem Längsschnitt eine Schutzvorrichtung,
- Fig. 2: eine Seitenansicht der Ausführungsform nach Fig. 1
- Fig. 3: eine Ansicht der Vorrichtung nach den Fig. 1 und 2 mit in die Schutzstellung verschobenem Schutzelement,
- Fig. 4: eine abgewandelte Vorrichtung nach den Fig. 1 und 2 mit einer Nadelkappe,
- Fig. 5: eine andere Ausführungsform eines Griffteiles in Verbindung mit einer Spritze,
- Fig. 6: eine abgewandelte Ausführungsform der Vorrichtung nach Fig. 5
- Fig. 7: eine Ausfübrungsform in Verbindung mit einem mit Flügeln versehenen Nadelhalter,
- Fig. 8: eine Schnittansicht der Ausführungsform nach Fig. 7,
- Fig. 9: eine Stimansicht in Fig. 7,
- Fig. 10: eine Draufsicht auf eine Ausführungsform nach Fig. 7 mit Nadelkappe,
- Fig. 11: eine Ansicht der Nadelkappe nach Fig. 10,
- Fig. 12: eine Draufsicht auf eine Ausführungsform nach Fig. 7 mit einer abgewandelten Nadelkappe,
- Fig. 13: eine Seitenansicht der Nadelkappe nach Fig. 12,
- Fig. 14: einen Längsschnitt durch eine Nadelkappe,
- Fig. 15: eine Schnittansicht durch eine andere Ausführungsform mit gekrümmter Nadel,
- Fig. 16: eine andere Nadelkappe bei der Ausführungsform nach Fig. 15,
- Fig. 17: eine Ausführungsform mit verformbarem Griffteil in der Ausgangsstellung,
- Fig. 18: das Griffteil von Fig. 17 in der gestreckten Stellung,
- Fig. 19: eine weitere Ausführungsform eines verformbaren Griffteils in der Ausgangsstellung, und
- Fig. 20: das Griffteil von Fig. 19 in der ausgefahrenen Stellung.

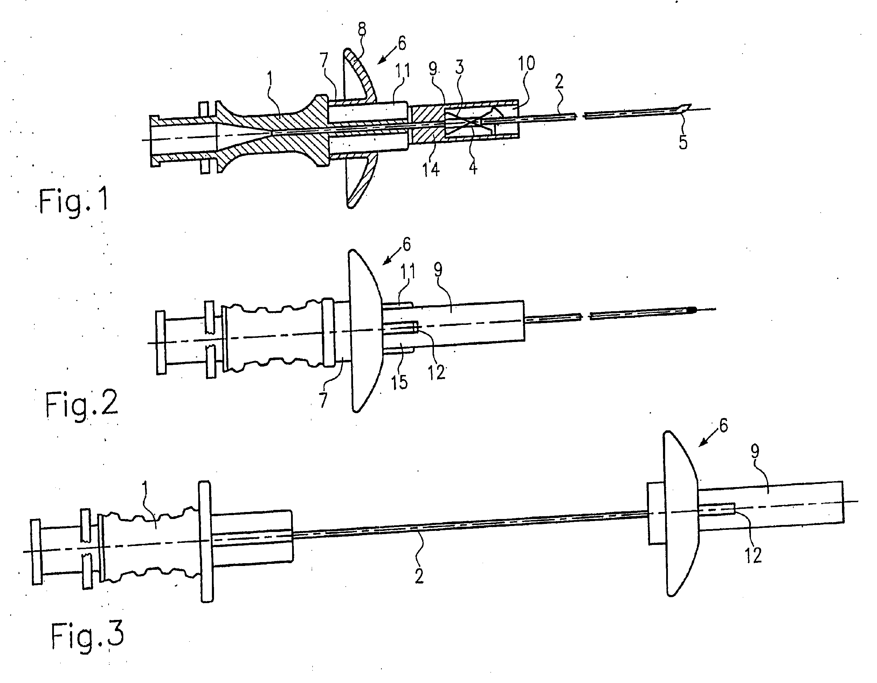

Fig. 1 zeigt einen Nadelhalter 1, in dem eine Nadel 2 befestigt ist. Auf dem Schaft der Nadel 2 ist ein Schutzelement 3 in Form eines Federclips mit sich kreuzenden Armen angeordnet. Mit 4 ist eine Hülse bezeichnet, die mit dem Schutzelement 3 längs des Nadelschaftes verschiebbar ist. Bei dem dargestellten Ausführungsbeispiel ist die Spitze 5 der Nadel entsprechend einer Epidural-Nadel oder einer Huber-Nadel gekrümmt ausgebildet, so dass die Hülse 4, die einen kleineren Durchmesser als die Krümmung an der Nadelspitze aufweist, und mit ihr das Schutzelement 3 nicht über die Nadelspitze hinaus verschoben werden kann.

Zwischen Nadelhalter 1 und Schutzelement 3 ist ein Griffteil 6 angeordnet, das am proximalen Ende einen hohlzylindrischen Abschnitt 7 aufweist, an dem ein radial abstehender Schild 8 ausgebildet ist. Auf der Vorderseite des Schildes 8 ist ein zylindrischer Abschnitt 9 ausgebildet, dessen distales Ende hohl ausgebildet ist. In dem Hohlraum 10 ist in der Bereitstellung nach Fig. 1 das Schutzelement 3 angeordnet, das durch Verschieben des Griffteiles 6 nach vorne zur Nadelspitze 5 verschoben werden kann, während mit der anderen Hand der Nadelhalter 1 gehalten wird. Hierbei übergreifen die abgewinkelten Enden der sich kreuzenden Arme des Schutzelementes 3 die Nadelspitze 5, so dass eine Verletzung der Bedienungsperson durch die Nadelspitze verhindert wird.

Der Nadelhalter 1 weist am distalen Ende radial abstehende Rippen 11 auf, auf denen der hohlzylindrische Abschnitt 7 des Griffteiles 6 geführt ist. Zwischen dem zylindrischen Abschnitt 9 mit kleinerem Außendurchmesser und dem hohlzylindrischen Abschnitt 7 mit größerem Außendurchmessers sind in dem Griffteil 6 Schlitze 12 ausgebildet, durch welche die vorderen Enden der Rippen 11 des Nadelhalters 1 radial vorstehen, wie Fig. 2 zeigt.

Der mit dem Hohlraum 10 versehene zylindrische Abschnitt 9 des Griffteils 6 weist einen vollzylindrischen Abschnitt 14 zwischen den Schlitzen 12 und dem Hohlraum 10 auf, in dessen zentrischer Bohrung die Nadel 2 geführt ist. Zwischen den Schlitzen 12 des Griffteils 6 ist der zylindrische Abschnitt 9 über Stege 15 mit dem Schild 8 bzw. dem hohlrylindrischen Abschnitt 7 einstückig verbunden.

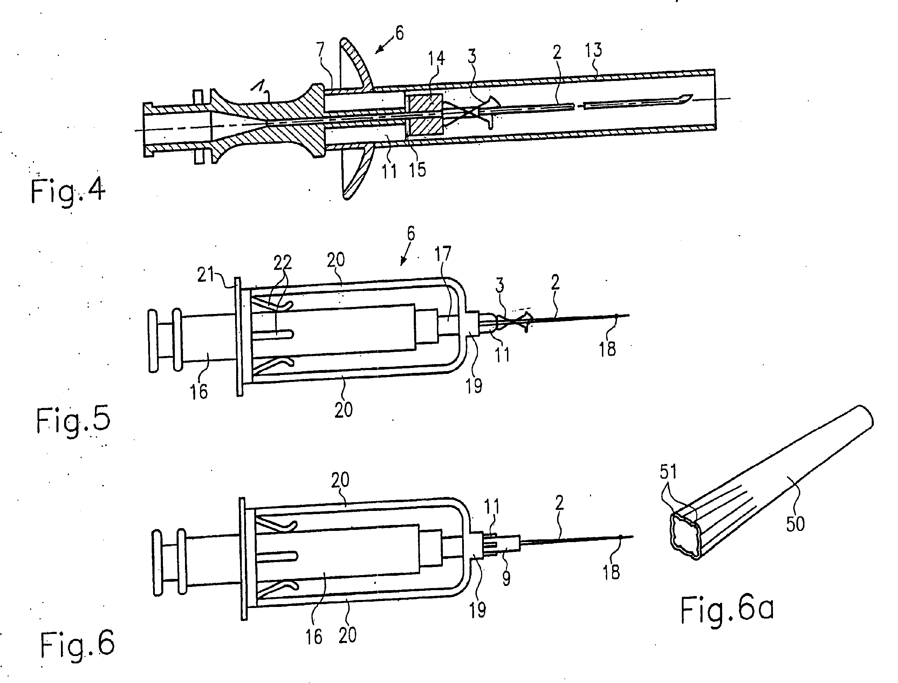

Diese über den Außenumfang des zylindrischen Abschnittes 9 des Griffteiles 6 vorstehenden Rippen 11 dienen zum Aufstecken einer Nadelkappe 13, die in Fig. 4 gezeigt ist. Diese Nadelkappe 13 wird für die Lagerung und die Handhabung der Vorrichtung verwendet. Sie kann unmittelbar vor Gebrauch der Injektionsnadel vom Nadelhalter 1 abgezogen werden, um die Nadel freizulegen, ohne dass dadurch das Griffteil 6 und das Schutzelement 3 bewegt wird, weil die Nadelkappe 13 durch die Rippen 11 in einem radialen Abstand von dem Abschnitt 9 des Griffteils 6 gehalten wird.

Aufgrund des kleineren Durchmessers am Abschnitt 14 gegenüber dem größeren Durchmesser an den Rippen 11 kann die Nadelkappe 13, die aus einem Schlauchstück mit durchgehend gleichem Durchmesser besteht, nicht auf dem Abschnitt 14 fehlpositioniert, sondern nur auf die Rippen 11 aufgesteckt werden. Hierdurch wird verhindert, dass ein Eingriff der Nadelkappe 13 mit einem Abschnitt des Griffteils 6 unbeabsichtigt hergestellt wird. Die Nadelkappe 13 kann durch Extrudieren eines Schlauches kostengünstig gefertigt werden, wobei ein Stück eines solchen Schlauches die Nadelkappe 13 bildet.

Nach Abnehmen der Nadelkappe 13 kann in der Bereitstellung nach den Fig. 1 und 2 eine Injektion ausgeführt werden, worauf beim Zurückziehen der Nadel mit einer Hand am Nadelhalter 1 mit der anderen Hand das Griffteil 6 am Abschnitt 7 gehalten wird, so dass durch die Relativbewegung zwischen Griffteil 6 und Nadel 2 das Schutzelement 3 in die Schutzstellung an der Nadelspitze verschoben wird. Diese ausgefahrene Stellung des Griffteiles 6 ist in Fig. 3 wiedergegeben.

Das Schutzelement 3 ist in dem Hohlraum 10 des Griffteils 6 lose angeordnet, so dass das Griffteil 6 aus der Stellung in Fig. 3 ohne weiteres zurückgezogen werden kann, während das Schutzelement in der Schutzstellung an der Nadelspitze verbleibt. Der Hohlraum 10 im zylindrischen Abschnitt 9 schützt das Schutzelement 3 nach Abziehen der Nadelkappe 13.

Fig. 4 zeigt eine bevorzugte Ausführungsform des Griffteils 6, wobei der hohlzylindrische Abschnitt am distalen Ende des Griffteils 6 weggelassen ist, so dass der vollzylindrische Abschnitt 14 das distale Ende des Griffteils 6 bildet. Nach Abziehen der Nadelkappe 13 von den Rippen 11 liegt in Fig. 4 das Schutzelement 3 frei.

Bei der Ausführungsform nach Fig. 1 und 2 dient der hohlzylindrische Abschnitt 7 am Griffteil 6 dazu, die Finger der das Griffteil haltenden Hand vor einer Berührung mit dem Nadelschaft zu schützen, wenn die Nadel zurückgezogen wird.

Bei einer anderen Ausgestaltung des Nadelhalters 1 kann dieser hohlzylindrische Abschnitt 7 hinter dem Schild 8 vergrößert ausgebildet sein.

Das Griffteil 6 wird - wie auch der Nadelhalter 1- zweckmäßigerweise aus Kunststoff gefertigt.

Fig. 5 zeigt eine abgewandelte Ausführungsform eines Griffteils 6 in Verbindung mit einer Spritze 16, an der über einen Nadelhalter 17, der als Kanülenansatz ausgebildet ist, eine Injektionsnadel 2 fest angebracht ist. Bei dieser Ausführungsform ist vor der Nadelspitze ein Wulst 18 auf dem Außenumfang der Nadel ausgebildet, an dem die Rückwand des Schutzelementes 3 in der Schutzstellung zum Anliegen kommt. Anstelle eines Wulstes 18 können durch eine Nadelquetschung auch diametral gegenüberliegende noppenartige Vorsprünge ausgebildet werden.

Das Griffteil 6 weist einen zylindrischen Abschnitt 19 auf, der in der Ausgangsstellung nach Fig. 5 auf dem Nadelhalter 17 geführt ist. Von diesem zylindrischen Abschnitt 19 erstrecken sich bei dem dargestellten Ausführungsbeispiel an diametral gegenüberliegenden Stellen zwei Bügel 20 in einem Abstand vom Spritzenumfang in proximale Richtung. Die Enden dieser Bügel 20 sind an einem Ringkörper 21 angeformt, von dem aus sich elastische Finger 22 radial nach innen erstrecken. Die freien Enden dieser elastischen Finger 22 liegen auf dem Au-βenumfang der Spritze 16 an.

Aufgrund der elastischen Finger 22 zwischen Griffteil 6 und Außenumfang der Spritze 16 kann das Griffteil 6 für unterschiedlich große Spritzendurchmesser verwendet werden, z. B. können Spritzen mit einem Volumen von 1 ml bis 10 ml in das gleiche Griffteil eingesetzt werden. Hierdurch ist eine große Auswahl von Spritzen möglich, die mit der gleichen Nadel verwendet werden können.

Auch bei der Ausführungsform nach Fig. 5 sind am vorderen Ende des als Kanülenansatz ausgebildeten Nadelhalters radial abstehende Rippen 11 ausgebildet werden, die als Sitz für eine Nadelkappe dienen. Durch den Innenumfang des zylindrischen Abschnitts 19 wird das Schutzelement 3, dessen Rückwand über den Querschnitt der Rippen 11 vorsteht, nach vorne in die Schutzstellung verschoben.

Fig. 6 zeigt eine Ausführungsform des Griffteils 6, bei der an dem zylindrischen Abschnitt 19 ein weiterer zylindrischer Abschnitt 9 angeformt ist, in dessen Hohlraum 10 das Schutzelement 3 aufgenommen wird. Wie bei der Ausführungsform nach den Fig. 1 und 2 sind zwischen zylindrischem Abschnitt 19 und Abschnitt 9 in Achsrichtung verlaufende Schlitze ausgebildet, durch die die am Nadelhalter bzw. Kanülenansatz 17 ausgebildeten Rippen 11 zur Aufnahme der Nadelkappe 13 vorstehen.

Fig. 6a zeigt in perspektivischer Ansicht eine durch Spritzgießen ausgebildete Nadelkappe 50, deren distales Ende geschlossen sein kann, während das proximale Ende auf dem Innenumfang entsprechend der Anzahl der Rippen 11 Rillen oder Nuten 51 aufweist, die beim. Aufstecken der Nadelkappe auf den Nadelhalter 17 mit den Rippen 11 in Eingriff treten, so dass durch Verdrehen der aufgesteckten Nadelkappe 50 auch der Nadelhalter 17 verdreht werden kann. Zwischen Nadelhalter 17 und Spritze 16 wird üblicherweise ein Gewindeeingriff vorgesehen, so dass durch Verdrehen der Nadelkappe 50 der Nadelhalter 17 auf die Spritze 16 aufgeschraubt werden kann.

Es ist üblich, mittels einer Nadel mit relativ großem Durchmesser Flüssigkeit in die Spritze anzusaugen und dann die Nadel gegen eine Nadel mit einem relativ kleinen Durchmesser auszuwechseln, um eine Infusion am Patienten auszuführen. Bei der Ausführungsform nach dem Fig. 5 und 6 kann die Nadel ohne weiteres ausgewechselt werden.

Die beschriebene Ausgestaltung ermöglicht eine Betätigung mit einer Hand, wenn der Spritzeninhalt injiziert ist, wobei die Spritze 16 mit zwei Fingern gehalten und die Nadel aus der Haut des Patienten gezogen wird, während gleichzeitig sich ein Finger der Hand an dem am proximalen Ende liegenden Ringkörper 21 abstützt.

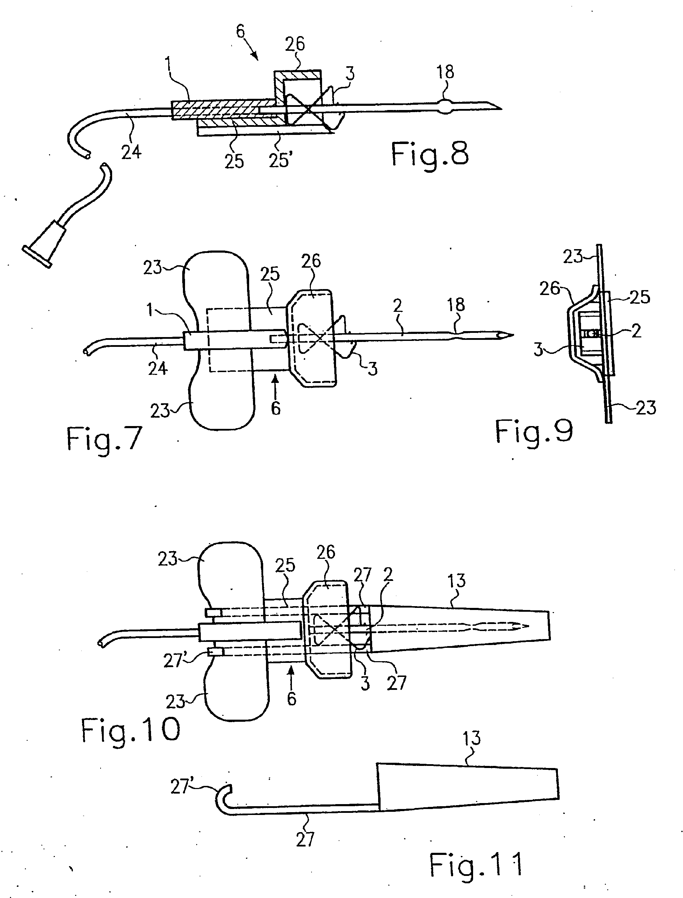

Fig. 7 zeigt in einer Draufsicht einen mit seitlich abstehenden Flügeln 23 versehenen Nadelhalter 1, an den ein Verbindungsschlauch 24 angeschlossen ist. Zwischen dem auf dem Nadelschaft angeordneten Schutzelement 3 und Nadelhalter 1 ist ein Griffteil 6 mit einem haubenförmigen Abschnitt 26 angeordnet, das wegen des flachen Injektionswinkels (Fig. 8) zweckmäßigerweise ein Flächenstück 25 zur Auflage auf der Haut des Patienten aufweist, das auf der Auflageseite beispielsweise mit einer Klebeschicht zur besseren Halterung auf der Haut versehen sein kann. Vorzugsweise wird ein Schaummaterial 25' auf der Auflageseite vorgesehen. Der von dem vorderen Ende des Flächenstücks 25 vorstehende Haubenabschnitt 26 des Griffteils 6 überdeckt wenigstens teilweise das Schutzelement 3. Das Flächenstück 25 bzw. das weiche Auflageteil 25' dient auch als Abstandhalter für das Schutzelement 3 von der Haut des Patienten. Bei dem Ausführungsbeispiel nach Fig. 8 erstreckt sich der weiche Auflageteil 25' über das Flächenstück 25 unter den Haubenabschnitt 26, so dass das Schutzelement 3 nicht auf der Haut des Patienten aufliegt.

Der mit Flügeln 23 versehene Nadelhalter 1 wird für Veneninfusionen verwendet, wobei üblicherweise eine dünne Nadel verwendet wird. Die Flügel 23 sind relativ groß und flexibel. Sie werden zusammengedrückt, wenn die Nadel in einem sehr flachen Winkel in die Haut eingeführt wird. Ein auf der Klebeschicht an der Auflagefläche aufgebrachtes, nicht dargestelltes Schutzpapier sollte nicht abgezogen werden, bis die Nadel in der Vene eingeführt ist. Nachdem die Nadel in die Vene eingeführt ist, werden die Flügel 23 auf der Haut des Patienten flach gelegt und mit einem Klebeband gehalten. Auch das Griffteil 6 kann mittels eines Klebebandes festgelegt werden, wobei der haubenförmige Abschnitt 26 einen Kontakt zwischen Schutzelement 3 und Klebeband verhindert. Beim Zurückziehen der Nadel nach Entfernen des Klebebandes vom Nadelhalter bleibt zunächst das Griffteil 6 mit dem Schutzelement 3 in seiner Stellung. Nachdem die zurückgezogene Nadelspitze durch das Schutzelement 3 sicher abgedeckt ist, wobei die Vorsprünge 18 an der Nadel das Schutzelement 3 an der Nadelspitze fixieren, kann auch das Griffteil 6 von der Haut des Patienten abgenommen werden.

Fig. 7 und 8 zeigen die Vorrichtung in der Bereitstellung zum Einführen der Nadel. Wenn die mit einer Klebeschicht versehene Auflage 25 am Griffteil verwendet wird, handelt es sich um ein passives System.

Fig. 9 zeigt eine Ansicht des Griffteils 6 von rechts in Fig. 7. Die Flügel 23 dienen als Auflagefläche für den Nadelhalter 1, nachdem die Infusionsnadel eine gewisse Zeit in der eingeführten Stellung verbleiben muss.

Fig. 10 und 11 zeigen bei einem Aufbau nach den Fig. 7 bis 9 eine Nadelkappe 13, die mit zwei beabstandeten Haltebügeln 27 versehen ist, die über ein abgebogenes freies Ende 27' an den Flügeln 23 eingehängt werden, wie Fig. 10 zeigt. Bei dieser Ausführungsform liegt das proximale Ende der Nadelkappe 13 zweckmäßigerweise am Vorderende des Schutzelementes 3 an, wie Fig. 10 zeigt, damit das Schutzelement 3 in seiner Bereitstellung gehalten wird.

Es ist aber auch möglich, am proximalen Ende der Nadelkappe 13 einen haubenförmigen Ansatz vorzusehen, der auf der Vorderseite des haubenförmigen Abschnitts 26 anliegt.

Bei den beschriebenen Ausführungsformen ist jeweils ein Schutzelement in der Form eines Federclips mit sich kreuzenden Armen wiedergegeben. Es kann aber auch eine andere Ausführungsform eines Schutzelementes in Verbindung mit dem Griffteil 6 verwendet werden.

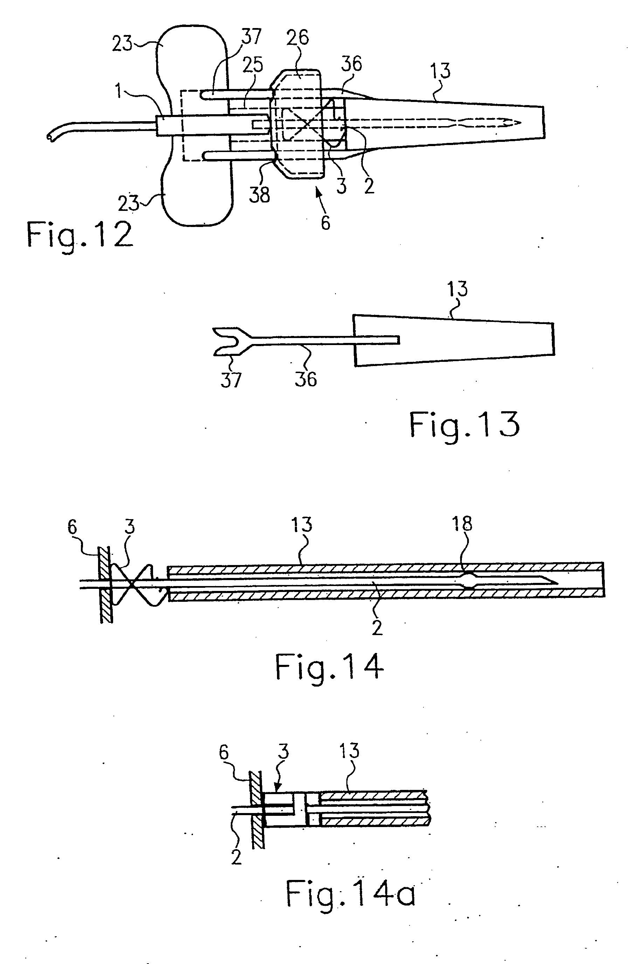

Fig. 12 zeigt eine weitere Ausführungsform der Vorrichtung nach den Fig. 7 bis 9. Die Nadelkappe 13 ist hierbei anstelle der Einhängebügel 27 auf den beiden Seiten mit jeweils einer Verlängerungsstrebe 36 versehen, die am freien Ende einen gabelförmigen Abschnitt 37 zum Einstecken an den Flügeln 23 des Nadelhalters aufweist (Fig. 13). Diese beiden beabstandeten Streben 36 erstrecken sich durch entsprechend groß ausgebildete Öffnungen 38 in dem Haubenabschnitt 26 des Griffteils 6, so dass die gabelförmigen Steckabschnitte 37 ohne weiteres durch diese Öffnungen 38 abgezogen werden können. Beim Abziehen der Nadelkappe 13 wird der Nadelhalter 1 gehalten, wobei das Griffteil 6 nicht bewegt wird.

Fig. 14 zeigt einen Längsschnitt durch eine Nadelkappe 13, deren proximales Ende am Schutzelement 3 anliegt. Die Nadelkappe ist rohrförmig ausgebildet, wobei die durch Quetschung ausgebildete Durchmesservergrößerung 18 an der Nadel 2 als Abstandhalter für die Nadelkappe 13 dient. Eine solche Nadelkappe kann durch Extrudieren oder Spritzgießen hergestellt werden. Es ist auch möglich, auf dem Innenumfang der Nadelkappe einen Wulst oder Noppen anzuformen, die am Nadelschaft aufliegen und die Nadel im Wesentlichen konzentrisch in der Nadelkappe führen. Die Nadelkappe 13 wird hierbei durch Reibung an den Vorwölbungen 18 auf der Nadel 2 gehalten.

Nach einer weiteren Ausgestaltung kann die Nadelkappe nach dem Aufstecken auf die Nadel durch Wärme und Druck bzw. durch Schrumpfen auf der Nadel festgelegt werden.

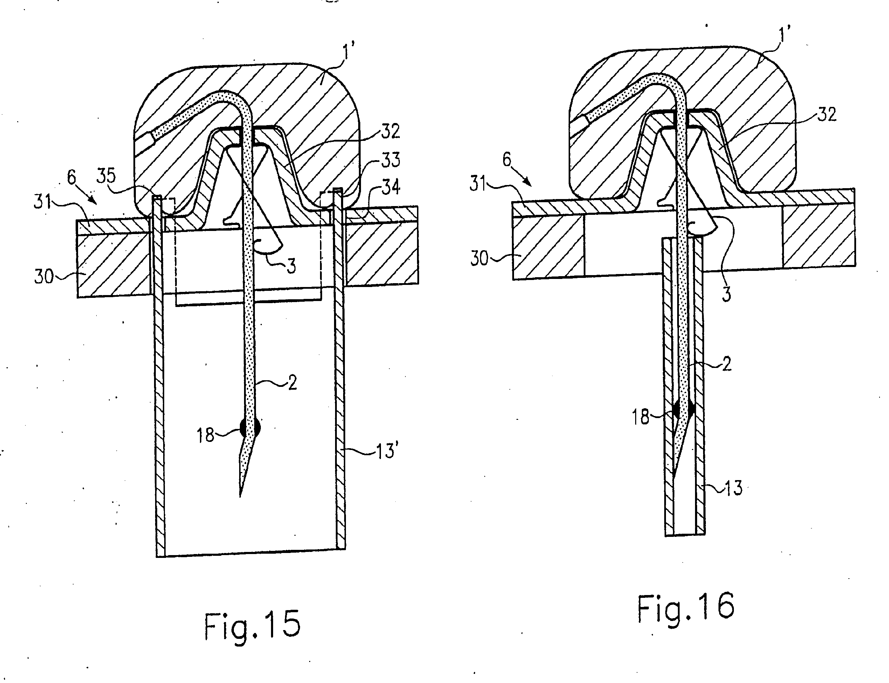

Fig. 15 zeigt eine Ausgestaltung in Verbindung mit einer Huber-Nadel 2, die mittels eines abgebogenen Abschnitts in einem Nadelhalter 1' gehalten ist und zum senkrechten Einführen bei einer Injektion vorgesehen ist. Mit 30 ist ein vorzugsweise aus Schaumstoffmaterial bestehendes Auflageteil bezeichnet, das mit einer Klebefläche zur besseren Fixierung auf der Haut des Patienten versehen ist. Zwischen dem Auflageteil 30 und dem Nadelhalter 1' ist ein schildförmiges Griffteil 6 angeordnet, das über einen flanschartigen Bereich 31 auf dem Auflageteil 30 aufliegt und sich über einen topfförmigen Mittelabschnitt 32 in eine entsprechende Vertiefung des Nadelhalters 1' erstreckt. In diesem topfförmigen Mittelteil 32 ist das Schutzelement 3 angeordnet.

Beim Herausziehen der Nadel wird das Griffteil 6 auf dem Auflageteil 30 gehalten, während der Nadelhalter 1' abgezogen wird. Hierbei wird das Schutzelement 3 zur Nadelspitze verschoben, bis es an der Nadelkröpfung 18 zum Anliegen kommt, während gleichzeitig die beiden sich kreuzenden Arme des Schutzelementes 3 die Nadelspitze übergreifen und abdecken. ' Hierauf kann das Griffteil 6 vom Auflageteil 30 oder zusammen mit diesem abgenommen werden: Griffteil 6 und Auflageteil 30 können über eine Klebeschicht auch miteinander verbunden sein.

Die Seitenwände des topfförmigen Mittelteils 32 sind vorzugsweise kegelförmig geneigt, so dass das Griffteil 6 selbst nicht abgezogen, sondern nur gedrückt werden kann.

Fig. 15 zeigt eine Nadelkappe 13' mit einem rohrförmigen Abschnitt, an dessen proximalem Ende diametral gegenüberliegende Wandabschnitte 33 vorstehen, die über teilkreisförmige Schlitze 34 in dem Flansch 31 des Griffteils 6 in entsprechend teilkreisförmige Nuten 35 im Nadelhalter 1' eingesteckt sind. Die gebogenen Wandabschnitte 33 sind lose durch die gebogenen Schlitze 34 im Flansch 31 des Griffteils 6 geführt und mit Preßsitz in die Nuten 35 des Nadelhalters 1' eingesteckt.

Wie bei den anderen Ausführungsformen einer Nadelkappe 13 kann auch die Nadelkappe 13' in Fig. 15 an der distalen Seite geschlossen ausgebildet sein.

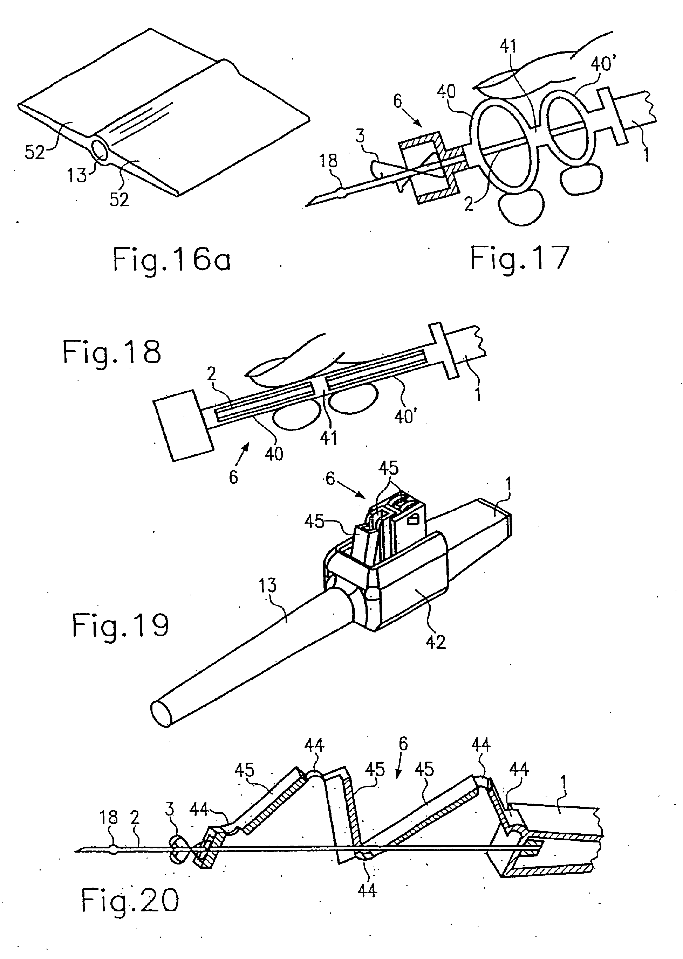

Fig. 16 zeigt eine Ausgestaltung mit Huber-Nadel 2 entsprechend Fig. 15, wobei eine Nadelkappe 13 mit kleinerem Innendurchmesser auf die Nadel 2 aufgeschoben ist. Die Nadelkappe entspricht im Wesentlichen der nach Fig. 14, wobei die Nadelkappe 13 durch Reibung an dem abgewinkelten Vorderende auf der Nadel gehalten wird. Diese Nadelkuppe 13 in Fig. 16 kann mit radial abstehenden und diametral gegenüberliegenden Flächenabschnitten 52 versehen sein, durch welche die Handhabung verbessert und die schlauchförmige Nadelkappe 13 versteift wird. Fig. 16a zeigt in perspektivischer Ansicht eine solche Nadelkappe 13 mit diametral gegenüberliegenden Flächenabschnitten 52.

Fig. 17 und 18 zeigen eine Ausführungsform, bei der das Griffteil 6 einen verformbaren Abschnitt aufweist, mittels dem das distale Ende des Griffteils, an dem das Schutzelement 3 anliegt, in Richtung der Schutzposition an der Nadelspitze verschoben werden kann, indem der verformbare Abschnitt verformt wird. Bei dem Ausführungsbeispiel nach Fig. 17 und 18 sind zwei Paare von formbaren Bügeln 40 und 40' an dem Griffteil 6 ausgebildet, die mit den Fingern zusammengedrückt werden können, so dass sie aus der gebogenen Stellung in Fig. 17 in eine gestreckte Stellung in Fig. 18 überführt werden. Die beiden verformbaren Bügelpaare 40 und 40' sind durch einen Hülsenabschnitt 41 miteinander verbunden. Es ist auch möglich, zwischen den beiden Bügelpaaren 40 und 40' ein Element einzusetzen, das durch Druck mittels der Finger die beiden verformbaren Bügel 40 und 40' in die gestreckte Stellung nach Fig. 17 überführt.

Fig. 19 und 20 zeigen eine weitere Ausführungsform eines verformbaren Griffteils 6, wobei Fig. 19 das Griffteil im zusammengefalteten Zustand in der Bereitstellung wiedergibt. Die Nadelkappe 13 ist am proximalen Ende mit einem Aufnahmeabschnitt 42 versehen, der zusammengefaltete Abschnitte 45 des Griffteils 6 aufnimmt, das zwischen Nadelhalter 1 und dem in Fig. 19 nicht dargestellten, im Aufnahmeabschnitt 42 angeordneten Schutzelement 3 angeordnet ist.

Fig. 20 zeigt in schematischer Darstellung das Griffteil 6 in teilweise ausgefahrenem Zustand, nachdem die Nadelkäppe 13 abgenommen ist und eine Injektion ausgeführt wurde. Hierauf werden die über Gelenk- bzw. Scharnierabschnitte 44 miteinander verbundenen steifen Abschnitte 45 des Griffteils 6, die teilweise auf der Nadel 2 geführt sind, längs der Nadel verschoben und ausgerichtet, wobei das Schutzelement 3 nach vorne zur Nadelspitze verschoben wird, bis es mit der Nadelkröpfung 18 in Eingriff tritt und die Nadelspitze abdeckt.

Die Ausführungsformen nach den Fig. 1 bis 16 haben gegenüber den Ausführungsformen nach den Fig. 17 bis 20 den Vorteil, dass eine größere Kanülenlänge in der Bereitstellung zur Verfügung steht, weil das Schutzelement 3 unmittelbar am Nadelhalter anliegt, während bei den Ausführungsformen nach den Fig. 17 bis 20 ein aufwendigerer Aufbau des Griffteils 6 zwischen Schutzelement 3 und Nadelhalter 1 vorgesehen ist, wodurch die zur Verfügung stehende Kanülenlänge eingeschränkt wird. Hierbei ist auch die Ausführungsform nach den Fig. 19 und 20 vorteilhafter hinsichtlich der Länge der zur Verfügung stehenden Kanüle als die Ausführungsform nach den Fig. 17 und 18, weil durch die Faltung der Abschnitte 45 eine kompaktere Anordnung möglich ist, wie Fig. 19 im Verhältnis zu Fig. 17 zeigt. Anstelle der Faltabschnitte in Fig. 20 kann auch ein Scherenmechanismus zwischen Schutzelement und Nadelhalter vorgesehen werden, um auf engem Raum Elemente unterzubringen, mittels denen das Schutzelement weit ausfahrbar ist.

Bei allen Ausführungsformen wird als Schutzelement 3 bevorzugt ein Nadelclip aus Metall verwendet, dessen sich kreuzende Arme von gegenüberliegenden Seiten eines proximalen Wandabschnitts ausgehen, der ein Loch für.den Durchtritt der Nadel aufweist, wobei der Lochdurchmesser kleiner ist als die maximale Querabmessung der Nadel an der Quetschung 18, so dass der Nadelclip durch den im Durchmesser vergrößerten Abschnitt 18 in der Schutzstellung an der Nadelspitze gehalten wird. Die sich kreuzenden Arme, die beiderseits der Nadel 2 verlaufen, wie Fig. 14a zeigt, weisen am distalen Ende einen etwa auf die Breite der Rückwand verbreiterten Endabschnitt auf, der in der Ausgangsstellung auf dem Außenumfang der Nadel unter elastischer Vorspannung aufliegt und bei Erreichen der Nadelspitze durch Federwirkung in die Schutzstellung bewegt wird, in der die beiden verbreiterten Endabschnitte die Nadelspitze übergreifen. Hierzu sind die distalen Enden der Arme, wie die Seitenansichten zeigen, in Längsrichtung etwas versetzt zueinander bzw. die Arme unterschiedlich lang, so dass sichergestellt ist, dass die beiden abgewinkelten Endabschnitte der Arme die Nadelspitze übergreifen. Zumindest am längeren Arm ist der Endabschnitt am freien Rand nach innen gebogen, um die Abdeckung der Nadelspitze auch dann zu gewährleisten, wenn versucht werden sollte, den Nadelclip aus der Schutzstellung auf der Nadel zurückzuschieben, wobei sich der nach innen abgebogene Endabschnitt an der Nadelspitze verhakt. Der Nadelclip kann insgesamt sehr kompakt ausgebildet werden und nur etwa 7 mm lang sein.

## Patentansprüche

1. Schutzvorrichtung für eine Injektions- bzw. Infusionsnadel, umfassend
- einen Nadelhalter (1) am proximalen Ende der Nadel,
- einen im Durchmesser vergrößerten Abschnitt (18) am Nadelschaft proximal von der Nadelspitze,
- ein Schutzelement (3) für die Nadelspitze; wobei das Schutzelement (3) aus einer Bereitstellung entlang dem Nadelschaft in die Schutzstellung verschiebbar ist, in der das Schutzelement (3) durch den im Durchmesser vergrößerten Abschnitt (18) der Nadel daran gehindert ist, über die Nadelspitze hinaus versehoben zu werden,
- ein Griffteil (6) zum Verschieben des Schutzelementes (3), welches zwischen Schutzelement (3) und Nadelhalter (1) angeordnet ist, wobei das Griffteil (6) einen Hohlraum (10) zur Aufnahme des Schutzelementes (3) aufweist, sodass das Schutzelement (3) ohne direktes Anfassen in die Schutzstellung gebracht werden kann, und
das Griffteil (6) am proximalen und am distalen Ende jeweils eine Öffnung aufweist, wobei das Schutzelement (3) größer ist als die Öffnung am proximalen Ende, sodass das Griffteil (6) daran gehindert ist, über das Schutzelement (3) und über die Nadelspitze hinaus verschoben zu werden, **dadurch gekennzeichnet, dass** der im Durchmesser vergrößerte Abschnitt (18) eine Nadelquetschung ist, und das Schutzelement zwei sich kreuzende Arme mit abgewinkelten Enden aufweist, die die Nadelspitze in der Schutzstellung übergreifen.

2. Schutzvorrichtung nach Anspruch 1, wobei der Hohlraum (10) das distale Ende des Schutzelementes (3) überdeckt.

3. Schutzvorrichtung nach Anspruch 1 oder 2, wobei das Schutzelement (3) vollständig im Hohlraum (10) des Griffteils (6) aufgenommen ist.

4. Schutzelement nach Anspruch 1, wobei die Arme des Schutzelementes (3) federnd ausgebildet sind.

5. Schutzvorrichtung nach einem der vorhergehenden Ansprüche, wobei ein Arm des Schutzelementes (3) länger ausgebildet ist als der andere Arm.

6. Schutzvorrichtung nach Anspruch 1, wobei das Griffteil (6) aus Kunststoff gefertigt ist.

7. Schutzvorrichtung nach Anspruch 1, wobei die Nadelquetschung (18) diametral gegenüberliegende Vorsprünge am Nadelschaft aufweist.

8. Schutzvorrichtung nach Anspruch 1, wobei der Nadelhalter (1) und das Griffteil (6) in der Bereitstellung sich überlappen.

9. Schutzvorrichtung nach Anspruch 1, wobei das Griffteil (6) einen zylindrischen Abschnitt (9) aufweist.

10. Schutzvorrichtung nach Anspruch 1, wobei die Wände des Hohlraums (10) im Griffteil die Arme des Schutzelementes (3) vollständig umgeben.

11. Schutzvorrichtung nach einem der vorhergehenden Ansprüche, wobei das Griffteil (6) länger ist als das Schutzelement (3).

12. Schutzvorrichtung nach Anspruch 1, wobei das Griffteil (6) einen radial abstehenden Schild (8) aufweist.

13. Schutzvorrichtung nach Anspruch 1, wobei das Griffteil (6) Schlitze (12) zur Aufnahme von radial abstehenden Rippen (11) am Nadelhalter (1) aufweist.

14. Schutzvorrichtung nach einem der vorhergehenden Ansprüche, wobei eine Nadelkappe (13) zum Schutz der Nadelspitze während der Lagerung und des Transports abnehmbar am Nadelansatz (1) angebracht ist.

15. Schutzvorrichtung nach Anspruch 13 oder 14, wobei die Nadelkappe (13) an den Rippen (11) des Nadelhalters (1) abnehmbar angebracht ist.

## Claims

1. A protective device for a hypodermic or infusion needle, comprising
- a needle holder (1) at the proximal end of the needle,
- a section (18) with enlarged diameter at the needle shaft proximal of the needle tip,
- a protective element (3) for the needle tip, wherein the protective element (3) is movable along the needle shaft from a ready position to a protective position, in which the protective element (3) is prevented by the needle section (18) with the enlarged diameter from being displayed beyond the needle tip,
- a grip part (6) for moving the protective element (3), which is arranged between the protective element (3) and the needle holder (1), wherein the grip part (6) comprises a cavity (10) for receiving the protective element (3), so that the protective element (3) can be brought into the protective position without being touched directly, and
the grip part (6) comprising an opening at the proximal and the distal end, wherein the protective element (3) is larger than the opening at the proximal end, such that the grip part (6) is prevented from being displaced beyond the protective element (3) and the needle tip,
**characterized in that**
the section (18) with enlarged diameter is a needle crimp, and that
the protective element comprises two intersecting arms with angled ends which overlap the needle tip in the protective position.

2. The protective device according to claim 1, wherein the cavity (10) covers the distal end of the protective element (3).

3. The protective device according to claim 1 or 2, wherein the protective element (3) is completely received in the cavity (10) of the grip part (6).

4. The protective element according to claim 1, wherein the arms of the protective element (3) are formed in a resilient manner.

5. The protective device according to any of the preceding claims, wherein one arm of the protective element (3) is formed longer than the other arm.

6. The protective device according to claim 1, wherein the grip part (6) is made of a plastic.

7. The protective device according to claim 1, wherein the needle crimp (18) comprises diametrically opposite protrusions on the needle shaft.

8. The protective device according to claim 1, wherein the needle holder (1) and the handle part (6) overlap each other in the ready position.

9. The protective device according to claim 1, wherein the grip part (6) comprises a cylindrical section (9).

10. The protective device according to claim 1, wherein the walls of the cavity (10) in the grip part completely surrounds the arms of the protective element (3).

11. The protective device according to any of the preceding claims, wherein the grip part (6) is longer than the protective element (6).

12. The protective device according to claim 1, wherein the grip part (6) comprises a radially projecting shield (8).

13. The protective device according to claim 1, wherein the grip part (6) comprises slits (12) to accommodate radially projecting ribs (11) on the needle holder (1).

14. The protective device according to any of the preceding claims, wherein a needle cap (13) is removably attached to the needle holder (1) to protect the needle tip during storage and transportation.

15. The protective device according to claim 13 or 14, wherein the needle cap (13) is removably attached to the ribs (11) of the needle holder (1).

## Revendications

1. Dispositif de protection pour aiguille d'injection ou de perfusion, comprenant
- un porte-aiguille (1) à l'extrémité proximale de l'aiguille,
- une section (18) au diamètre accru sur la tige de l'aiguille proximale de la pointe de l'aiguille,
- un élément de protection (3) pour la pointe de l'aiguille, dans lequel l'élément de protection (3) est déplaçable d'une position prête à l'utilisation le long de la tige de l'aiguille dans la position de protection, dans lequel on empêche l'élément de protection (3) au moyen de la section (18) au diamètre accru d'être déplacé au-delà de la pointe de l'aiguille,
- une partie poignée (6) pour déplacer l'élément de protection (3), qui est disposé entre l'élément de protection (3) et le porte-aiguille (1), dans lequel la partie poignée (6) présente une cavité (10) pour la réception de l'élément de protection (3), de sorte que l'élément de protection (3) puisse être amené dans la position de protection sans contact direct et
- la partie poignée (6) ayant un orifice à chacune de ses extrémités proximale et distale dans lequel l'élément de protection (3) est plus gros que l'orifice sur l'extrémité proximale, de sorte que la partie poignée (6) ne peut pas être déplacée au-delà de l'élément de protection (3) et de la pointe de l'aiguille,
**caractérisé en ce que**
la section (18) au diamètre accru est un pincement d'aiguille et **en ce que** l'élément de protection présente deux bras qui se croisent avec des extrémités coudées, qui dépassent la pointe de l'aiguille dans la position de protection.

2. Dispositif de protection selon la revendication 1, dans lequel la cavité (10) recouvre l'extrémité distale de l'élément de protection (3).

3. Dispositif de protection selon la revendication 1 ou 2, dans lequel l'élément de protection (3) est logé totalement dans la cavité (10) de la partie poignée (6).

4. Dispositif de protection selon la revendication 1, dans lequel les bras de l'élément de protection (3) sont conçus de façon élastique.

5. Dispositif de protection selon l'une quelconque des revendications précédentes, dans lequel un bras de l'élément de protection (3) est conçu pour être plus long que l'autre bras.

6. Dispositif de protection selon la revendication 1, dans lequel la partie poignée (6) est composée de plastique.

7. Dispositif de protection selon la revendication 1, dans lequel pincement d'aiguille (18) présente des saillies diamétralement opposées au niveau de la tige de l'aiguille.

8. Dispositif de protection selon la revendication 1, dans lequel le porte-aiguille (1) et la partie poignée (6) se chevauchent dans la position prête à l'utilisation.

9. Dispositif de protection selon la revendication 1, dans lequel la partie poignée (6) présente une section cylindrique (9).

10. Dispositif de protection selon la revendication 1, dans lequel les parois de la cavité (10) dans la partie poignée entourent complètement les bras de l'élément de protection (3).

11. Dispositif de protection selon l'une quelconque des revendications précédentes, dans lequel la partie poignée (6) est conçue pour être plus longue que l'élément de protection (3).

12. Dispositif de protection selon la revendication 1, dans lequel la partie poignée (6) présente un panneau en saillie au plan radial (8).

13. Dispositif de protection selon la revendication 1, dans lequel la partie poignée (6) présente des fentes (12) pour la réception de nervures (11) en saillie au plan radial sur le porte-aiguille (1).

14. Dispositif de protection selon l'une quelconque des revendications précédentes, dans lequel un capuchon d'aiguille (13) est prévu amovible sur le raccord d'aiguille (1), pour la protection de la pointe d'aiguille pendant le stockage et le transport.

15. Dispositif de protection selon la revendication 13 ou 14, dans lequel le capuchon d'aiguille (13) est prévu amovible sur les nervures (11) du porte-aiguille (1).
